# FASCICULE DE BREVET EUROPEEN

(11) **EP 3 927 384 B1**
(45) Date de publication et mention de la délivrance du brevet: **11.11.2026**
(21) Numéro de dépôt: 20705368.7
(22) Date de dépôt: 19.02.2020
(51) Int. Cl.: A61L 24/04

(54) **COLLES CHIRURGICALES A BASE DE MONOMERES COMPRENANT UNE FONCTION PHOSPHATE**
CHIRURGISCHE KLEBSTOFFE AUF DER GRUNDLAGE VON MONOMEREN MIT PHOSPHATFUNKTION
SURGICAL GLUES BASED ON MONOMERS COMPRISING A PHOSPHATE FUNCTION

(30) Priorité: 21.02.2019 FR 1901771
(43) Date de publication de la demande: 29.12.2021
(73) Titulaire: Cohesives, 21000 Dijon (FR); Centre national de la recherche scientifique, 75016 Paris (FR); Université de Haute Alsace, 68093 Mulhouse Cedex (FR)
(72) Inventeur: AUBIN, Ariane, 68100 MULHOUSE (FR); LALEVEE, Jacques, 68100 MULHOUSE (FR); SCHWEBELEN, Jean-Philippe, 68520 BURNHAUPT-LE-HAUT (FR); PERRIN, Bertrand, 21000 DIJON (FR)
(74) Mandataire: Oudin, Stéphane
(86) Numéro de dépôt international: PCT/EP2020/054387
(87) Numéro de publication internationale: WO 2020/169681

(56) Documents cités:
- WO-A1-2016/011028
- WO-A2-2008/082929
- US-A1- 2017 209 617

## Description

### Domaine technique

La présente invention concerne notamment des compositions à utiliser comme adhésif chirurgical, produit d'étanchéification chirurgical, pansement hémostatique et pansement cutané. Plus particulièrement, la présente invention concerne des compositions destinées à être utilisées dans un procédé:
- en tant qu'adhésif chirurgical pour l'adhésion d'un matériau à un tissu biologique,
- pour l'adhésion de tissus biologiques entre eux,
- pour l'adhésion d'une colle ou d'une substance à la surface d'un tissu biologique,
- en tant qu'étanchéificateur chirurgical,
- pour obturer ou colmater les orifices crées par une suture par fil ou par agrafe ou par une résection tissulaire (hémostase, aérostase, lymphostase par exemple),
- pour obturer un orifice, une incision ou une déchirure dans un tissu biologique,
- en tant qu'hémostatique pour arrêter un saignement, seul ou en complément techniques conventionnelles d'hémostase comme la suture, la compression ou l'électrocoagulation,
- en tant que pansement sur un tissu biologique pour recouvrir et protéger une plaie,
Ces compositions peuvent également être utilisées :
- pour renforcer un tissu biologique,
- pour fixer et stabiliser un tissu biologique.

### Etat de la technique

Un certain nombre de techniques chirurgicales mettent en œuvre des colles chirurgicales. Ces dernières sont principalement utilisées pour aider à obtenir une hémostase chirurgicale. Toutefois, l'efficacité des colles chirurgicales dans cette indication est controversée et d'autres utilisations comme pour l'aérostase ne montrent pas de meilleurs résultats.

Par ailleurs, les colles chirurgicales ont de très faibles propriétés adhésives et ne peuvent donc pas être utilisées en tant qu'adhésif ni comme suture chirurgicale. L'application des colles chirurgicales se fait la plupart du temps directement sur le tissu, sans préparation de la surface de collage. La pénétration dans les tissus est faible ou inexistante ce qui entraine un collage de mauvaise qualité. Les déposants ont constaté que les colles actuelles ne collent pas et ne pénètrent pas dans les tissus.

Afin de répondre à ce problème, des colles chirurgicales de faibles viscosités ont été proposées (WO2016/185153A1). Ces colles chirurgicales pénètrent plus facilement dans les tissus ce qui permet d'obtenir un collage de meilleure qualité. Toutefois, ces colles chirurgicales nécessitent des concentrations importantes de monomères. Par ailleurs, leur nature chimique peut engendrer des brûlures sur le site d'apposition.

Le document WO2008082929 décrit des compostions adhésives pour les tissus durs (dent, os) comprenant 16% de MDP, 44% de méthacrylates et 10% d'agent de réticulation.

En conséquence, la présente invention se propose de fournir un nouveau type de colles chirurgicales. Les compositions et le procédé selon l'invention permettent d'obtenir un collage efficace et résistant avec des quantités de monomères inférieures à celle des colles de l'art antérieur. Par ailleurs, contrairement aux colles de l'art antérieur, les colles chirurgicales selon l'invention ne provoquent pas de brûlures de la peau.

### Résumé de l'invention

La présente invention concerne des compositions pour utilisation en tant qu'adhésif chirurgical dans une méthode de traitement chirurgical ou thérapeutique du corps humain ou animal pour l'adhésion d'un matériau à un tissu biologique non minéralisé, pour l'adhésion de tissus biologiques non minéralisés entre eux, pour l'adhésion d'une colle ou d'une substance à la surface d'un tissu biologique non minéralisé, en tant qu'étanchéificateur chirurgical, pour obturer ou colmater les orifices crées par une suture par fil ou par agrafe ou par une résection tissulaire, pour obturer un orifice, une incision ou une déchirure dans un tissu biologique non minéralisé, en tant qu'hémostatique pour arrêter un saignement, en tant que pansement sur un tissu biologique non minéralisé pour recouvrir et protéger une plaie, pour renforcer un tissu biologique non minéralisé, pour fixer et stabiliser un tissu biologique non minéralisé caractérisé en ce que lesdites compositions comprennent entre 10 et 60% en masse par rapport à la masse totale de la composition de 10-MDP (C₁₄H₂₇O₆P) ou de MEP (C₁₂H₁₉O₈P).

Les déposants ont pu mettre en évidence que la présence d'une fonction phosphate ou phosphonate dans ledit monomère permettait d'obtenir une composition présentant des capacités adhésives et une innocuité supérieure à celles des compositions comprenant un monomère chimiquement équivalent comprenant une fonction méthacrylate mais pas de fonction phosphate.

Dans le cadre de la présente invention le terme « monomère polymérisable » entend désigner un monomère dont la polymérisation peut être initiée par un activateur physique ou chimique.

Selon un mode de réalisation préféré, la polymérisation est initiée sous l'effet d'un rayonnement UV. De manière préférée, ledit rayonnement UV a une longueur d'onde comprise entre de 200 nm à 400 nm, encore plus préférentiellement entre 300 nm à 400 nm et tout à fait préférentiellement entre 350 nm et 400 nm.

Selon un autre mode de réalisation préféré, la polymérisation est initiée sous l'effet d'un rayonnement de longueur d'onde comprise entre 400 nm et 500 nm.

Selon un mode de réalisation préféré, la polymérisation est amorcée par un activateur chimique.

Le polymère obtenu après polymérisation du monomère est préférentiellement un polymère biocompatible.

Préférentiellement la viscosité de la composition selon l'invention est inférieure à 200 mPa.s à 20°C.

La viscosité de la composition peut notamment être mesurée par un viscosimètre à chute de bille selon la norme DIN53015.

Selon un mode de réalisation préféré, ladite viscosité est inférieure à 120 mPa.s à 20°C.

Selon un mode de réalisation encore plus préféré, ladite viscosité est inférieure à 50 mPa.s à 20°C.

Selon un mode de réalisation tout à fait préféré, ladite viscosité est inférieure à 20 mPa.s à 20°C.

Selon un mode de réalisation préféré, la composition selon l'invention n'est pas un hydrogel.

Selon un mode de réalisation préféré, ledit monomère a une masse molaire comprise entre 250 et 500g.mol-1.

Selon un mode de réalisation préféré, ledit monomère a une concentration comprise entre 10 et 60% en masse par rapport à la masse totale de la composition.

Selon un mode de réalisation préféré, ladite composition comprend en outre entre 4% et 30%, en masse par rapport à la masse totale de la composition, d'un premier agent de réticulation.

L'homme du métier est apte à choisir l'agent de réticulation le plus adapté en fonction du monomère polymérisable utilisé.

Selon un mode de réalisation préféré, ledit premier agent de réticulation comprend une fonction acrylate ou une fonction méthacrylate.

Selon un mode de réalisation préféré, ledit premier agent de réticulation est choisi dans le groupe comprenant les acrylates et méthacrylates multi-fonctionnels comprenant notamment le 1,6-hexanediol di acrylate, le triméthylolpropane tri acrylate, le 1,2-éthylène glycol di acrylate, le pentaérythritol tétracrylate, le I 3-(acryloyloxy)-2-hydroxypropyl méthacrylate (AHM), le diméthacrylate d'uréthane (UDMA), le diméthacrylate d'hexanediol (HDDMA), le diméthacrylate de triéthylèneglycol (TEGDMA), et les mélanges de ceux-ci.

Selon un autre mode de réalisation préféré, ledit premier agent de réticulation est choisi dans le groupe comprenant les acrylates multi-fonctionnels comprenant notamment l'hexanediol diméthylacrylate (HDDMA), l'éthylène glycol diméthylacrylate (EGDMA), le butanediol diacrylate (BDDA), le poly(éthylène glycol) diacrylate (PEGDA) et les mélanges de ceux-ci.

Selon un mode de réalisation préféré, ledit premier agent de réticulation est l'UDMA et est présent à une concentration comprise entre 4.5% et 5% en masse par rapport à la masse totale de la composition.

Selon un mode de réalisation préféré, ledit premier agent de réticulation est le TEGDMA et est présent à une concentration comprise entre 25% et 30% en masse par rapport à la masse totale de la composition.

Selon un autre mode de réalisation préféré, ladite composition comprend en outre entre 30% et 90%, en masse par rapport à la masse totale de la composition, d'un second agent de réticulation.

Selon un autre mode de réalisation préféré ledit second agent de réticulation est choisi dans le groupe comprenant les poly(éthylène glycol) acrylate multifonctionels (fonctionnalité 2-4) comprenant notamment le SR415, SR610 et l'Ebecryl 11, et les mélanges de ceux-ci.

Selon un autre mode de réalisation préféré, ledit second agent de réticulation est choisi dans le groupe comprenant les résines de nature acrylique uréthane aliphatique difonctionnelles comprenant notamment l'Ebecryl4491, l'Ebecryl 230, l'Ebecryl 271, l'Ebecryl 2221 et le CN9002. Selon un autre mode de réalisation préféré, ledit second agent de réticulation est choisi dans le groupe comprenant les résines de nature acrylique uréthane aromatique difonctionnelles comprenant notamment l'Ebecryl 210, et les mélanges de ceux-ci.

Selon un autre mode de réalisation préféré, ledit second agent de réticulation est un choisi dans le groupe comprenant les résines époxy acrylate difonctionnelles comprenant notamment EB3639.

Selon un autre mode de réalisation préféré, ledit second agent de réticulation est choisi dans le groupe comprenant les résines acryliques comprenant notamment Ebecryl 8296, Ebecryl 8232, Ebecryl ODA, Ucecoat 6569, et les mélanges de ceux-ci.

Par souci de clarté, il est précisé que certains des monomères polymérisables selon l'invention peuvent agir comme agent de réticulation et que dans ce dernier cas les concentrations préférées décrites ci-dessus ne sont pas pertinentes.

Selon un mode de réalisation préféré, ladite composition comprend en outre un co-monomère.

Selon un mode de réalisation encore plus préféré ledit co-monomère est choisi dans le groupe comprenant les polybutadiene diacrylates et les monomères mono ou di-fonctionnels tels que tert-butyl acrylate, n-butyl acrylate, lauryl acrylate, lauryl methacrylate, méthyle acrylate, stéaryl methacrylate, hydroxyethyl acrylate, hydroxyethyl methacrylate, acide acrylique, acide méthacrylique, 2-ethoxyethyl methacrylate, 2-ethylhexyl acrylate monomer, 2-ethylhexyl methacrylate, 2-phenyloxyethyl methacrylate, 1-(acryloyloxy)-3-(methacryloyloxy)-2-propanol, di(ethylene glycol) ethyl ether acrylate, ethyl acrylate, ethylene glycol methyl ether acrylate, ethylene glycol phenyl ether acrylate, methyl acrylate, 3-(trimethoxysilyl)propyl methacrylate, methyl methacrylate, poly(ethylene glycol) methyl ether acrylate (Mn de 200 à 10 000 g/mol), Triéthylene glycol diméthacrylate, tert-butyl méthacrylate, triethylene glycol monoethylether methacrylate, 3-(tris(trimethylsilyloxy)silyl)propyl methacrylate.

Selon un mode de réalisation préféré, ledit co-monomère est présent dans la composition selon l'invention a une concentration comprise entre 1 et 50% en masse par rapport à la masse totale de la composition.

Selon un mode de réalisation préféré la composition selon l'invention comprend un photo-amorceur. L'homme du métier choisira en fonction du spectre d'émission de la lampe utilisée le photoamorceur le plus adapté.

Le photoamorceur peut être notamment choisi parmi : l'oxyde de biacylphosphine (BAPO), le Bis(.eta.5-2,4-cylcopentadien-1-yl)-bis(2,6-difluoro-3-(1H-pyrrol-1-yl)- phenyl) titaniuml (Irgacure 784), l' 1-[4-(2-Hydroxyethoxy)-phenyl]-2-hydroxy-2-methyl-1-propane-1-one (Irgacure 2959), l'oxyde de 2,4,6-triméthylbenzoyl-phénylphosphinate (TPO-L), le 2,4,6-trimethylbenzoyldiphenylphosphine oxide (TPO), le 2,2-diméthoxyphényl-2-acétophénone (DMPA), la camphorquinone ou 4,4'-bis(diethylamino)benzophenone cette dernière associée à la N-Phenylglycine (NPG), l'Ethyl-4-(dimethylamino)benzoate (EDB), la N-Diisopropylethylamine (DIPEAN) ou au 4-(Dimethylamino)benzonitrile (DMABN).

Avantageusement le photoamorceur est utilisé à une concentration comprise entre 0,2 et 10% en masse.

Selon un mode de réalisation préféré, ledit photo-amorceur est le TPO-L.

Selon un autre mode de réalisation préféré, la polymérisation est amorcée par un activateur chimique et plus particulièrement par le peroxyde de benzoyle associé à la N-Phenylglycine (NPG), l'Ethyl-4-(dimethylamino)benzoate (EDB), la N-Diisopropylethylamine (DIPEAN) ou au 4-(Dimethylamino)benzonitrile (DMABN).

Avantageusement l'activateur chimique est utilisé à une concentration comprise entre 0,5 et 3% en masse.

Selon un mode de réalisation préféré, ladite composition ne comprend que ledit monomère et un photoamorceur ou que ledit monomère, un premier ou second agent de réticulation et un photoamorceur.

Selon un mode de réalisation de l'invention, ladite composition comprend un solvant et encore plus préférentiellement ledit solvant est de l'eau. Selon un autre mode de réalisation préféré, ledit solvant est un alcool et tout à fait préférentiellement de l'éthanol ou l'isopropanol.

Selon un autre mode de réalisation préféré, ladite composition est dépourvue de solvant.

Selon un mode de réalisation préféré de l'invention, ladite composition comprend un tampon. Selon un mode de réalisation encore plus préféré, ledit tampon est un tampon basique et de manière tout à fait préférée ledit tampon basique est choisi dans le groupe comprenant le KOH, le carbonate de guanidine, le Ca(OH)₂, le K₂CO₃, le 2-amino-2-methyl-1-propanol (AMP), le Na₂CO₃, le résorcinol et le NaOH.

L'homme du métier est à même de choisir les concentrations de tampon les plus adaptées à cette utilisation. Selon un mode de réalisation tout à fait préféré de l'invention, la composition comprend une concentration de KOH comprise entre 0 et 8% en masse.

Selon un mode réalisation préféré de l'invention, ladite composition comprend entre 0.1 et 5% en masse de photoamorceur, entre 30 et 90% en masse d'un second agent de réticulation, entre 1 et 50% en masse de monomère polymérisable comprenant une fonction phosphate et une fonction méthacrylate selon l'invention et entre 1 et 50% en masse de co-monomère.

Les compositions décrites dans les tableaux ci-dessous sont plus particulièrement préférées (les quantités sont en pourcentage de la masse totale de la composition.

**[Tableau 1]**

| TPO-L | UDMA | TEGDM A | MDP | tBuA | BM | LMA | LA | KOH, 5M dans ethanol |
|---|---|---|---|---|---|---|---|---|
| 1 à 2 | 4,5 à 5.5 | 0 | 55 à 65 | 34.83 | 0.01 à 0.05 | 0 | 0 | 0 |
| 1 à 2 | 0 | 25 à 35 | 20 à 25 | 45.52 | 0.01 à 0.05 | 0 | 0 | 0 |
| 1 à 2 | 0 | 25 à 35 | 10 à 15 | 55.2 | 0.01 à 0.05 | 0 | 0 | 0 |
| 1 à 2 | 4,5 à 5.5 | 0 | 15 à 20 | 74.8 | 0.01 à 0.05 | 0 | 0 | 0 |
| 1 à 2 | 4,5 à 5.5 | 0 | 25 à 30 | 0 | 0.01 à 0.05 | 15 à 25 | 0 | 0 |
| 1 à 2 | 0 | 0 | 40 à 60 | 0 | 0 | 0 | 0 | |
| 1à2 | 4,5 à 5.5 | 0 | 25 à 30 | 24.71 | 0.01 à 0.05 | 0 | 5 | 5 à 15 |

**[Tableau 2]**

| TPO-L | MEP | tBuA | AA | BM | KOH 5m dans éthanol |
|---|---|---|---|---|---|
| 1 à 2 | 10 à20 | 80 à 90 | 0 | 0.01 à 0.05 | 0 |
| 1 à 2 | 20 à 30 | 70 à 80 | 0 | 0.01 à 0.05 | 0 |
| 1 à 2 | 3 à 7 | 60 à 70 | 15 à 25 | 0.01 à 0.05 | 5 à 15 |
| 1 à 2 | 3 à 7 | 20 à 30 | 25 à 35 | 0.01 à 0.05 | 5 à 15 |
| 1à2 | 3 à 7 | 75 à 85 | 5 à 15 | 0.01 à 0.05 | 3 à 7 |

Dans le cadre de la présente invention, le terme « comprend » entend signifier que la composition selon l'invention inclut les éléments cités. De manière préférée, la présente invention concerne des compositions comprenant uniquement les éléments cités à l'exclusion de tout autre.

La présente description concerne également un procédé (ne faisant pas partie de l'invention), non invasif, pour l'adhésion d'un matériau à un tissu biologique, pour l'adhésion de tissus biologiques entre eux, pour l'adhésion d'une colle ou d'une substance à la surface d'un tissu biologique , l'étanchéification chirurgical, pour obturer ou colmater les orifices crées par une suture par fil ou par agrafe ou par une résection tissulaire (hémostase, aérostase, lymphostase par exemple), pour obturer un orifice une incision ou une déchirure dans un tissu biologique, pour arrêter un saignement, pour recouvrir et protéger une plaie, pour renforcer un tissu biologique ou pour fixer et stabiliser un tissu biologique, remarquable en ce qu'il comprend les étapes :
- (i) enduire le tissu à traiter d'une composition selon invention
- (ii) laisser pénétrer la composition dans ledit tissu,
- (iii) induire la polymérisation de ladite composition.

Dans le cadre de la présente invention, le terme « tissu biologique » entend désigner préférentiellement les tissus biologiques non minéralisés.

Par souci de clarté, il est précisé que, dans le cadre de la présente invention, le terme « tissus biologique » n'entend pas désigner les os et les dents.

Le procédé est avantageusement non-invasif. Le terme « non-invasif » entend signifier que le procédé selon l'invention ne comprend aucune étape chirurgicale consistant à accéder au tissu à traiter. Ainsi, le procédé selon l'invention est mis en œuvre sur un tissu biologique directement accessible (e.g. la peau) ou préalablement rendu accessible par d'autres méthodes.

Selon un mode de réalisation préféré, ladite étape (iii) est réalisée à l'aide d'un rayonnement UV. Les caractéristiques du rayonnement UV mis en œuvre, notamment sa puissance et sa longueur d'onde, sont adaptées aux constituants de la composition, notamment à la nature du monomère polymérisable et à sa concentration dans la composition.

Selon un autre mode de réalisation préféré, ladite étape (iii) est réalisée à l'aide d'un rayonnement de lumière visible.

Selon un mode de réalisation préféré, ledit rayonnement UV a une longueur d'onde comprise entre 350 et 400 nm.

Selon un mode de réalisation préféré, ledit rayonnement UV a une puissance d'irradiance comprise entre 20mW/cm2 et 500 mW/cm2.

La présente description concerne également un ensemble (ne faisant pas partie de l'invention) de parties comprenant une composition selon l'invention et une source de rayonnement UV. De préférence la source de rayonnement UV de l'ensemble de parties peut émettre un rayonnement UV adapté pour polymériser et/ou aider la polymérisation et/ou accélérer la polymérisation du monomère polymérisable de la composition. La présente description concerne également un ensemble de parties (ne faisant pas partie de l'invention) comprenant une composition selon l'invention et un initiateur de polymérisation chimique.

Dans le cadre de la présente invention, le terme « source de rayonnement UV » fait référence à tout moyen artificiel apte à produire un rayonnement UV et plus particulièrement un rayonnement de longueur d'onde comprise entre 200 et 400 nm encore plus préférentiellement entre 300 nm à 400 nm et tout à fait préférentiellement entre 350 nm et 400 nm. De manière préférée ledit rayonnement UV est une puissance d'irradiance comprise entre 20mW/cm2 et 500 mW/cm2 et encore plus préférentiellement entre 50mW/cm2 et 150 mW/cm2.

De manière tout à fait préférée, ledit rayonnement UV est de longueur d'onde comprise entre 350 et 400 nm et de puissance comprise entre 50-200 mW/cm2.

### Description des modes de réalisation

### Matériels et méthodes

### Test de réaction cutanée

### Chez l'animal

Des compositions selon l'invention et des compositions contrôles ont été déposées sur des dos et des abdomens de lapins et de rats préalablement rasés et désinfectés.

La composition est déposée à la surface de la peau puis polymérisée à l'aide d'une source d'irradiation lumineuse.

La présence ou l'absence de réactions cutanées sont observées lors de la mise en place puis dans les jours qui suivent.

### Chez l'homme

Des compositions selon l'invention et des compositions contrôles ont été déposées sur la face antérieure de l'avant-bras.

La composition est déposée à la surface de la peau puis polymérisée par source de polymérisation.

La présence ou l'absence de réactions cutanées sont observées lors de la mise en place puis dans les jours qui suivent.

Chez l'homme et chez l'animal, les compositions suivantes ont été testées :

**[Tableau 3]**

| TPO-L (%) | UDMA (%) | TEGDM A (%) | tBuA (%) | MDP (%) | LMA (%) | BM (%) | AA |
|---|---|---|---|---|---|---|---|
| 1.5 | 5 | n | 34.8 | 58.7 | n | 0.02 | 0 |
| 1.5 | 0 | 29.98 | 45.52 | 23.01 | 0 | 0.02 | 0 |
| 1.5 | 5 | 0 | 34.8 | 0 | 58.7 | 0.02 | 0 |
| 1.5 | 0 | 29.98 | 45.52 | 0 | 23.01 | 0.02 | 0 |
| 1.5 | 4.98 | 0 | 34,83 | | | 0.02 | 5870 |

Dans le tableau 3, seules les compositions présentant entre 10 et 60% en masse de MDP par rapport à la masse totale de la composition font partie de l'invention. **Test de pelage** Des compositions selon l'invention et des compositions contrôles ont été déposées sur des échantillons de péricardes bovins. Cette étape est réalisée à 20°C. Lesdits échantillons de péricarde ont été soumis à un rayonnement UV de 395nm, pendant une durée de 45 s., afin de déclencher la polymérisation des monomères. La source de rayonnement a été placée à 10 cm du péricarde. Lesdits échantillons de péricarde ont été ensuite recouverts d'une compresse tissée 17 fils, cette dernière a ensuite reçu une solution de monomères identique à celle utilisée à l'étape précédente.

Les échantillons de péricarde ont été soumis à un rayonnement UV dans des conditions identiques à celles de l'étape précédente.

Le temps de repos de la compresse installée entre les mors de la machine de traction est d'une minute, la température au sein de l'échantillon est, au moment du lancement du test, de 30°C + ou - 4 °C.

### Résultats

### Test de réaction cutanée

Tous les tests effectués chez l'homme et l'animal avec des compositions à base d'acide acrylique ou d'acide méthacrylique provoquent une irritation cutanée et/ou de brûlures cutanées.

Au contraire, les tests effectués avec des compositions selon l'invention comprenant du MDP et du MEP (monomères ayant une fonction acrylate et une fonction phosphate) ont montré une absence de réactions cutanées apparentes.

Ces résultats indiquent sans aucun doute, qu'à concentration de monomères équivalente, les compositions selon l'invention sont moins agressives pour les tissus traités.

### Test de pelage

Sur tous les essais effectués, on observe que les compositions comprenant des monomères sans fonction phosphate ne permettent pas d'obtenir d'adhésion de bonnes qualités, c'est-à-dire que le pelage se fait à l'interface peau/bande de fibres enduites.

Au contraire, les compositions selon l'invention, comprenant une fonction phosphate et une fonction acrylique permettent d'obtenir, quelle que soit la concentration en monomères, une adhésion de bonne qualité mise en évidence par un pelage se faisant intégralement dans le tissu traité.

On observe ainsi, qu'à concentration équivalente et à structure chimique proche, la présence d'une fonction phosphate dans le monomère polymérisable utilisé dans la composition selon l'invention permet d'augmenter la résistance à la rupture et assure l'aspect cohésif par pénétration dans les couches superficielles du tissu à traiter (i.e. la résistance du collage).

## Revendications

1. Composition pour utilisation en tant qu'adhésif chirurgical dans une méthode de traitement chirurgical ou thérapeutique du corps humain ou animal pour l'adhésion d'un matériau à un tissu biologique non minéralisé, pour l'adhésion de tissus biologiques non minéralisés entre eux, pour l'adhésion d'une colle ou d'une substance à la surface d'un tissu biologique non minéralisé, en tant qu'étanchéificateur chirurgical, pour obturer ou colmater les orifices crées par une suture par fil ou par agrafe ou par une résection tissulaire, pour obturer un orifice, une incision ou une déchirure dans un tissu biologique non minéralisé, en tant qu'hémostatique pour arrêter un saignement, en tant que pansement sur un tissu biologique non minéralisé pour recouvrir et protéger une plaie, pour renforcer un tissu biologique non minéralisé, pour fixer et stabiliser un tissu biologique non minéralisé **caractérisé en ce que** lesdites compositions comprennent entre 10 et 60% en masse par rapport à la masse totale de la composition de 10-MDP (C₁₄H₂₇O₆P) ou de MEP(C₁₂H₁₉O₈P).

2. Composition selon la revendication précédente **caractérisée en ce qu'**elle comprend en outre entre 4% et 30%, en masse par rapport à la masse totale de la composition, d'un premier agent de réticulation.

3. Composition pour utilisation
selon la revendication 1 **caractérisée en ce qu'**elle comprend en outre entre 30% et 90%, en masse par rapport à la masse totale de la composition, d'un second agent de réticulation

4. Composition pour utilisation
selon l'une des revendications précédentes **caractérisée en ce qu'**elle comprend en outre un co-monomère.

5. Composition pour utilisation
selon la revendication précédente **caractérisée en ce que** ledit co-monomère est choisi dans le groupe comprenant les polybutadiene diacrylates et les monomères mono ou di-fonctionnels tels que tert-butyl acrylate, n-butyl acrylate, lauryl acrylate, lauryl methacrylate, méthyle acrylate, stéaryl methacrylate, hydroxyethyl acrylate, hydroxyethyl methacrylate, acide acrylique, acide méthacrylique, 2-ethoxyethyl methacrylate, 2-ethylhexyl acrylate monomer, 2-ethylhexyl methacrylate, 2-phenyloxyethyl methacrylate, 1-(acryloyloxy)-3-(methacryloyloxy)-2-propanol, di(ethylene glycol) ethyl ether acrylate, ethyl acrylate, ethylene glycol methyl ether acrylate, ethylene glycol phenyl ether acrylate, methyl acrylate, 3-(trimethoxysilyl)propyl methacrylate, methyl methacrylate, poly(ethylene glycol) methyl ether acrylate (Mn de 200 à 10 000 g/mol), Triéthylene glycol diméthacrylate, tert-butyl méthacrylate, triethylene glycol monoethylether methacrylate, 3-(tris(trimethylsilyloxy)silyl)propyl methacrylate.

6. Composition pour utilisation
selon la revendication précédente **caractérisée en ce que** ledit co-monomère a une concentration comprise entre 1 et 50% en masse par rapport à la masse totale de la composition.

7. Composition pour utilisation
selon l'une des revendications 1 à 5, **caractérisée en ce qu'**elle comprend en outre un photo-amorceur.

8. Composition pour utilisation
selon la revendication 6, **caractérisée en ce que** ledit photo-amorceur est choisi dans le groupe comprenant l'oxyde de biacylphosphine (BAPO), le Bis(.eta.5-2,4-cylcopentadien-1-yl)-bis(2,6-difluoro-3-(1H-pyrrol-1-yl)- phenyl) titaniuml (Irgacure 784), l' 1-[4-(2-Hydroxyethoxy)-phenyl]-2-hydroxy-2-methyl-1-propane-1-one (Irgacure 2959), l'oxyde de 2,4,6-triméthylbenzoyl-phénylphosphinate (TPO-L), le 2,4,6-trimethylbenzoyldiphenylphosphine oxide (TPO), le 2,2-diméthoxyphényl-2-acétophénone (DMPA), la camphorquinone ou 4,4'-bis(diethylamino)benzophenone cette dernière associée à la N-Phenylglycine (NPG), l'Ethyl-4-(dimethylamino)benzoate (EDB), la N-Diisopropylethylamine (DIPEAN) ou au 4-(Dimethylamino)benzonitrile (DMABN).

9. Composition pour utilisation
selon l'une des revendications 6 à 7, **caractérisée en ce que** ledit photo-amorceur est à une concentration comprise entre 0,2 et 10% en masse.

10. Composition pour utilisation
selon la revendication 1 **caractérisée en ce qu'**elle comprend entre 0.1 et 5% en masse de photoamorceur, entre 30 et 90% en masse d'un second agent de réticulation, entre 10 et 50% en masse de 10-MDP (C₁₄H₂₇O₆P) ou de MEP(C₁₂H₁₉O₈P) et entre 1 et 50% en masse de co-monomère.

## Patentansprüche

1. Zusammensetzung zur Verwendung als chirurgischer Klebstoff in einem Verfahren zur chirurgischen oder therapeutischen Behandlung des menschenlichen oder tierischen Körpers zur Haftung eines Materials an einem nicht mineralisiertem biologischen Gewebe, zur Haftung von nicht mineralisierten biologischen Geweben untereinander, zur Haftung eines Klebstoffs oder einer Substanz an der Oberfläche von nicht mineralisiertem biologischem Gewebe, als chirurgisches Dichtungsmittel, zum Verschließen oder Abdichten von Öffnungen, die durch Faden- oder Klammernaht oder durch Geweberesektion entstanden sind, zum Verschließen einer Öffnung, einer Inzision oder eines Risses in nicht mineralisiertem biologischem Gewebe, als Hämostasemittel zum Stoppen von Blutungen, als Verband auf nicht mineralisiertem biologischem Gewebe, um eine Wunde abzudecken und zu schützen, zur Verstärkung von nicht mineralisiertem biologischem Gewebe, zur Fixierung und Stabilisierung von nicht mineralisiertem biologischem Gewebe, **dadurch gekennzeichnet, dass** die Zusammensetzungen zwischen 10 und 60 Masse-%, bezogen auf die Gesamtmasse der Zusammensetzung, an 10-MDP (C₁₄H₂₇O₆P) oder MEP(C₁₂H₁₉O₈P) umfassen.

2. Zusammensetzung nach dem vorhergehenden Anspruch, **dadurch gekennzeichnet, dass** sie ferner zwischen 4 Masse-% und 30 Masse-%, bezogen auf die Gesamtmasse der Zusammensetzung, eines ersten Vernetzungsmittels umfasst.

3. Zusammensetzung zur Verwendung nach Anspruch 1, **dadurch gekennzeichnet, dass** sie ferner zwischen 30 Masse-% und 90 Masse-%, bezogen auf die Gesamtmasse der Zusammensetzung, eines zweiten Vernetzungsmittels umfasst.

4. Zusammensetzung zur Verwendung nach einem der vorhergehenden Ansprüche, **dadurch gekennzeichnet, dass** sie ferner ein Co-Monomer umfasst.

5. Zusammensetzung zur Verwendung nach dem vorhergehenden Anspruch, **dadurch gekennzeichnet, dass** das Co-Monomer aus der Gruppe ausgewählt ist, die Polybutadiendiacrylate und mono- oder difunktionale Monomere wie tert-Butylacrylat, n-Butylacrylat, Laurylacrylat, Laurylmethacrylat, Methylacrylat, Stearylmethacrylat, Hydroxyethylacrylat, Hydroxyethylmethacrylat, Acrylsäure, Methacrylsäure, 2-Ethoxyethylmethacrylat, 2-Ethylhexylacrylat-Monomer, 2-Ethylhexylmethacrylat, 2-Phenyloxyethylmethacrylat, 1-(Acryloyloxy)-3-(methacryloyloxy)-2-propanol, Di(ethylenglykol)ethyletheracrylat, Ethylacrylat, Ethylenglykolmethyletheracrylat, Ethylenglykolphenyletheracrylat, Methylacrylat, 3-(Trimethoxysilyl)propylmethacrylat, Methylmethacrylat, Poly(ethylenglykol)methyletheracrylat (Mn von 200 bis 10.000 g/mol), Triethylenglykoldimethacrylat, tert-Butylmethacrylat, Triethylenglykolmonoethylethermethacrylat, 3-(Tris(trimethylsilyloxy)silyl)propylmethacrylat umfasst.

6. Zusammensetzung nach einem der vorhergehenden Ansprüche, **dadurch gekennzeichnet, dass** das Co-Monomer eine Konzentration zwischen 1 und 50 Masse-%, bezogen auf die Gesamtmasse der Zusammensetzung, aufweist.

7. Zusammensetzung zur Verwendung nach einem der Ansprüche 1 bis 5, **dadurch gekennzeichnet, dass** sie ferner einen Photoinitiator umfasst.

8. Zusammensetzung für die Verwendung nach Anspruch 6, **dadurch gekennzeichnet, dass** der Photoinitiator aus der Gruppe ausgewählt ist, die Biacylphosphinoxid (BAPO), Bis(.eta.5-2,4-cylcopentadien-1-yl)-bis(2,6-difluor-3-(1H-pyrrol-1-yl)-phenyl)titan (Irgacure 784), 1-[4-(2-Hydroxyethoxy)-phenyl]-2-hydroxy-2-methyl-1-propan-1-on (Irgacure 2959), 2,4,6-Trimethylbenzoylphenylphosphinatoxid (TPO-L), 2,4,6-Trimethylbenzoyldiphenylphosphinat (TPO), 2,2-Dimethoxyphenyl-2-acetophenon (DMPA), Campherchinon oder 4,4'-bis(Diethylamino)benzophenon, wobei letzteres mit N-Phenylglycin (NPG) assoziiert ist, Ethyl-4-(dimethylamino)benzoat (EDB), N-Diisopropylethylamin (DIPEAN) oder 4-(Dimethylamino)benzonitril (DMABN) umfasst.

9. Zusammensetzung zur Verwendung nach einem der Ansprüche 6 bis 7, **dadurch gekennzeichnet, dass** der Photoinitiator eine Konzentration zwischen 0,2 und 10 Masse-% aufweist.

10. Zusammensetzung zur Verwendung nach Anspruch 1, **dadurch gekennzeichnet, dass** sie zwischen 0,1 und 5 Masse-% des Photoinitiators, zwischen 30 und 90 Masse-% eines zweiten Vernetzungsmittels, zwischen 10 und 50 Masse-% 10-MDP (C₁₄H₂₇O₆P) oder MEP(C₁₂H₁₉O₈P) und zwischen 1 und 50 Masse-% des Co-Monomers umfasst.

## Claims

1. Composition for use as a surgical adhesive in a method of surgical or therapeutic treatment of the human or animal body for adhesion of a material to a non-mineralised biological tissue, for adhesion of non-mineralised biological tissues to each other, for adhesion of a glue or substance to the surface of a non-mineralised biological tissue, as a surgical sealant, to plug or seal holes created by suture or staple or by tissue resection, to plug a hole, incision or tear in non-mineralised biological tissue, as a hemostatic agent to stop bleeding, as a dressing on non-mineralised biological tissue to cover and protect a wound, to reinforce non-mineralised biological tissue, to fix and stabilise a non-mineralised biological tissue, **characterised in that** said compositions comprise between 10 and 60% by mass relative to the total mass of the composition of 10-MDP (C₁₄ H₂₇ O₆ P) or MEP(C₁₂ H₁₉ O₈ P).

2. Composition according to the preceding claim, **characterised in that** it further comprises between 4% and 30%, by mass relative to the total mass of the composition, of a first crosslinking agent.

3. Composition for use according to claim 1, **characterised in that** it further comprises between 30% and 90%, by mass relative to the total mass of the composition, of a second crosslinking agent

4. Composition for use according to any one of the preceding claims, **characterised in that** it further comprises a co-monomer.

5. Composition for use according to the preceding claim **characterised in that** said co-monomer is selected from the group comprising polybutadiene diacrylates and mono or difunctional monomers such as tert-butyl acrylate, n-butyl acrylate, lauryl acrylate, lauryl methacrylate, methyl acrylate, stearyl methacrylate, hydroxyethyl acrylate, hydroxyethyl methacrylate, acrylic acid, methacrylic acid, 2-ethoxyethyl methacrylate, 2-ethylhexyl acrylate monomer, 2-ethylhexyl methacrylate, 2-phenyloxyethyl methacrylate, 1-(acryloyloxy)-3-(methacryloyloxy)-2-propanol, di(ethylene glycol) ethyl ether acrylate, ethyl acrylate, ethylene glycol methyl ether acrylate, ethylene glycol phenyl ether acrylate, methyl acrylate, 3-(trimethoxysilyl)propyl methacrylate, methyl methacrylate, poly(ethylene glycol) methyl ether acrylate (Mn from 200 to 10,000 g/mol), triethylene glycol dimethacrylate, tert-butyl methacrylate, triethylene glycol monoethylether methacrylate, 3-(tris(trimethylsilyloxy)silyl)propyl methacrylate.

6. Composition for use according to the preceding claim, **characterised in that** said co-monomer has a concentration between 1 and 50% by mass relative to the total mass of the composition.

7. Composition for use according to any one of claims 1 to 5, **characterised in that** it further comprises a photoinitiator.

8. Composition for use according to claim 6, **characterised in that** said photoinitiator is selected from the group comprising biacylphosphine oxide (BAPO), bis(.eta.5-2,4-cylcopentadien-1-yl)-bis(2,6-difluoro-3-(1H-pyrrol-1-yl)-phenyl) titanium l (Irgacure 784), 1-[4-(2-hydroxyethoxy)-phenyl]-2-hydroxy-2-methyl-1-propane-1-one (Irgacure 2959), 2,4,6-trimethylbenzoyl-phenylphosphinate (TPO-L) oxide, 2,4,6-trimethylbenzoyldiphenylphosphine oxide (TPO), 2,2-dimethoxyphenyl-2-acetophenone (DMPA), camphorquinone or 4,4'-bis(diethylamino)benzophenone, the latter combined with N-phenylglycine (NPG), ethyl-4-(dimethylamino)benzoate (EDB), N-diisopropylethylamine (DIPEAN) or 4-(dimethylamino)benzonitrile (DMABN).

9. Composition for use according to either claim 6 or 7, **characterised in that** said photoinitiator is at a concentration between 0.2% and 10% by mass.

10. Composition for use according to claim 1, **characterised in that** it comprises between 0.1 and 5% by mass of photoinitiator, between 30 and 90% by mass of a second crosslinking agent, between 10 and 50% by mass of 10-MDP (C₁₄ H₂₇ O₆ P) or MEP(C₁₂ H₁₉ O₈ P) and between 1 and 50% by mass of co-monomer.
